# FASCICULE DE BREVET EUROPEEN

(11) **EP 4 348 244 B1**
(45) Date de publication et mention de la délivrance du brevet: **21.05.2025**
(21) Numéro de dépôt: 22731750.0
(22) Date de dépôt: 24.05.2022
(51) Int. Cl.: G01N 21/95, G01N 33/08, A01K 43/00

(54) **PROCÉDÉ DE CONTRÔLE AU VOL D'OEUFS PLACÉS DANS DES CONTENANTS**
VERFAHREN ZUR KONTROLLE IM LAUFENDEM BETRIEB DER IN BEHÄLTER EINGELEGTEN EIER
METHOD FOR CONTROLLING ON THE FLY EGGS PLACED IN CONTAINERS

(30) Priorité: 01.06.2021 FR 2105779
(43) Date de publication de la demande: 10.04.2024
(73) Titulaire: Egg-Chick Automated Technologies, 29400 Landivisiau Cedex (FR)
(72) Inventeur: TRUBUIL, Laura, 29800 Plouedern (FR); LHARIDON, Devan, 29800 Plouedern (FR)
(74) Mandataire: Plasseraud IP
(86) Numéro de dépôt international: PCT/FR2022/050985
(87) Numéro de publication internationale: WO 2022/254123

(56) Documents cités:
- CN-A- 110 583 530
- US-A1- 2009 201 323

## Description

### Domaine technique

La présente invention concerne un procédé de contrôle au vol d'œufs placés dans des contenants, notamment pour le contrôle automatique de ces œufs sur une ligne de traitement à hautes cadences.

Elle concerne également un appareil pour la mise en œuvre d'un tel procédé de contrôle.

### Technique antérieure

Il est connu dans le domaine de l'aviculture, en particulier dans la production de poussins, d'utiliser les propriétés optiques des oeufs pour discriminer ces derniers (cf. par exemple US 2009/201323A1) et ignorer lors du traitement les œufs identifiés comme étant non susceptibles d'éclore et de donner naissance à un poussin.

Ces derniers sont essentiellement des oeufs infertiles ou des oeufs fécondés mais dont l'embryon de l'oeuf est mort ou encore malformé.

Cette discrimination est rendue nécessaire non seulement pour minimiser les pertes de vaccins lors de traitement in ovo, c'est-à-dire lors d'une injection par aiguille de vaccin au travers de la coquille de l'oeuf en vue de favoriser son éclosion et de prévenir l'apparition de maladies, mais également pour éviter l'explosion d'œufs pourris susceptibles de contaminer des œufs environnants qui sont viables dans le contenant, et le matériel d'injection pouvant servir à injecter ces œufs viables, ce qui risquerait également de contaminer ces derniers.

Notons que l'explosion d'œufs pourris est également susceptible de venir salir les écrans de protection des optiques utilisées dans la discrimination des œufs, le procédé associé à cette dernière étant communément appelé « mirage ».

Les salissures résultant de ces explosions peuvent nuire à la qualité de détection de l'état de certains œufs lorsqu'elles restent légères, voire empêcher une telle détection si les salissures sont plus importantes. La machine mise en œuvre pour réaliser le mirage, dénommée machine de mirage, doit dès lors être arrêtée pour assurer son nettoyage.

Il est également connu que certaines étapes de contrôle des œufs d'un contenant ne sont possibles qu'avec l'arrêt du contenant et/ou à une faible cadence de traitement.

Un tel arrêt du contenant, entraîné par un convoyeur, est couramment obtenu par l'introduction d'un bloqueur mécanique sur le chemin de déplacement de celui-ci ou encore par le simple arrêt du convoyeur.

Or, on observe qu'un arrêt brutal d'un contenant entraîne un brusque mouvement des œufs dans leur alvéole, ou cellule, lequel conduit à un désaxage de ces derniers, ou encore à un écartement de chaque œuf par rapport à la verticale passant par le centre de son alvéole correspondante.

Ce désaxage des œufs est bien entendu préjudiciable pour l'injection in ovo subséquente d'un vaccin, l'aiguille de l'injecteur n'étant alors plus nécessairement dirigée vers la chambre à air de l'oeuf correspondant.

Non seulement l'efficacité de la vaccination peut en être affectée, mais l'embryon risque également d'être tué.

Un tel arrêt occasionne également l'application de variations soudaines de vitesse et éventuellement de chocs sur les embryons, lesquels peuvent avoir des impacts négatifs sur leur vitalité.

De plus, l'arrêt de chaque contenant limite nécessairement la cadence maximale que peut atteindre la ligne de traitement associée.

Les cadences observées avec ces machines de l'état de l'art restent, par conséquent, faibles.

Il existe donc un besoin pressant pour un procédé de contrôle d'œufs placés dans les alvéoles de contenants, dont la conception originale permette de surmonter les inconvénients de l'art antérieur exposés ci-dessus.

### Objet de l'invention

La présente invention vise à pallier les inconvénients de l'art antérieur en proposant un procédé et un appareil de contrôle d'œufs placés dans des contenants, simples dans leur conception et dans leur mode opératoire, permettant un défilement continu des contenants, c'est-à-dire sans arrêt de ces derniers.

Un autre objet de la présente invention est un tel procédé et un tel appareil de contrôle autorisant des cadences extrêmement rapides, et à titre illustratif, supérieures à 90 000 œufs à l'heure.

Encore un objet de la présente invention est un tel procédé et un tel appareil de contrôle plus sûr pour les embryons des œufs, et par conséquent, favorisant l'éclosion de ces œufs.

Encore un autre objet de la présente invention est un tel procédé et un tel appareil de contrôle préservant l'orientation des œufs dans leur alvéole pour une injection in ovo ultérieure de meilleure qualité.

### Exposé de l'invention

A cet effet, l'invention concerne un procédé de contrôle au vol, sans contact, d'œufs placés dans des contenants. Selon l'invention,
- pendant le déplacement de ces contenants le long d'une ligne de transport, lesdits contenants étant espacés les uns des autres d'au moins une distance de séparation d minimale, on réalise les étapes suivantes:
- on déclenche un cycle d'acquisition de données à chaque passage d'une extrémité avale d'un contenant en une première position, déterminée par un premier capteur de position placé le long de ladite ligne de transport, les positions amont et aval étant considérées dans le sens de déplacement des contenants, puis
- pour un cycle d'acquisition de données d'un contenant, on détecte le passage de ladite extrémité avale dudit contenant en au moins une deuxième position déterminée par un deuxième capteur de position placé le long de ladite ligne de transport, un signal de déclenchement d'acquisition d'image thermique étant envoyé à une caméra thermique à chaque détection de ladite extrémité avale dudit contenant en au moins une deuxième position pour déclencher une prise d'une ou plusieurs images thermiques par ladite caméra thermique de la partie de contenant alors placée dans son champ de vision,
- lesdits deuxièmes capteurs de position étant agencés les uns par rapport aux autres pour assurer un contrôle de l'ensemble des œufs du contenant considéré par ladite caméra thermique lorsque plusieurs deuxièmes capteurs sont mis en œuvre,
- pendant le cycle d'acquisition de données dudit contenant, on réalise également en une troisième position, distincte desdites première et deuxième positions, une étape de mirage des œufs placés dans ledit contenant, selon laquelle on émet un flux lumineux en direction d'au moins un œuf à mirer et on analyse ensuite le flux lumineux passé à travers chaque œuf correspondant en fonction du taux de flux lumineux absorbé par l'oeuf, et
- lesdites données ainsi acquises des œufs d'un contenant étant associées à un identifiant unique de ce contenant.

La conception originale de ce procédé de contrôle au vol d'œufs autorise des cadences extrêmement rapides, typiquement supérieures à 90 000 œufs à l'heure et plus, tout en étant plus sûr pour les œufs.

On constate en effet que les œufs sont soumis à moins de mouvements, moins de chocs, et sont, par conséquent, mieux positionnés dans leur alvéole respective en vue de leur injection ultérieure.

En outre, ce procédé garantit avantageusement un contrôle un à un des contenants en mouvement de sorte que les images et signaux lumineux collectés ne peuvent être attribués qu'à un seul contenant à la fois. Ce procédé se révèle donc plus sûr dans le traitement des données à des cadences élevées.

Pour chaque capteur de position, on détecte le front montant du signal de mesure lié à la détection du passage de l'extrémité avale du contenant au droit de ce capteur. Bien entendu, on pourrait tout aussi bien détecter le front descendant du signal de mesure.

Bien qu'une seule caméra thermique soit préférée, le présent procédé pourrait, bien entendu, mettre en œuvre plusieurs caméras thermiques dont les champs de vision viendraient par exemple couvrir une dimension d'un contenant, telle que sa largeur.

Selon un mode de réalisation de ce procédé de contrôle au vol d'œufs placés dans des contenants, cette étape de mirage est réalisée :
- soit à un instant t₁ à compter de la détection de l'extrémité avale dudit contenant en ladite première position, t₁ étant inférieur au temps nécessaire à l'extrémité avale dudit contenant pour parvenir en ladite au moins une deuxième position,
- soit par la détection de ladite extrémité avale dudit contenant par un troisième capteur de position placé entre ledit premier capteur de position et ledit au moins un deuxième capteur de position le long de ladite ligne de transport des contenants.
Alternativement, cette étape de mirage est déclenchée par la détection du passage de ladite extrémité avale dudit contenant en une troisième position déterminée par un troisième capteur de position placé en aval d'une part du premier capteur de position et d'autre part dudit ou desdits deuxièmes capteurs de position, le long de ladite ligne de transport.

A titre d'exemple, cette étape de mirage comprend la focalisation d'un faisceau de lumière sur chaque œuf et la détection de la lumière ayant traversé chaque œuf correspondant. Les sources de lumières mises en œuvre pour émettre les faisceaux de lumière sont avantageusement des lasers, et encore mieux des diodes électroluminescentes (LEDs) émettant dans l'infrarouge.

De préférence, on traite automatiquement la lumière détectée et on utilise les données obtenues à partir de ce traitement pour traiter la ou les images obtenues par la caméra thermique

Ainsi, et de manière avantageuse, les œufs étant positionnés en rangées dans des alvéoles dans chaque contenant, on détermine par traitement des signaux obtenus lors de ladite étape de mirage, la ou les alvéoles vides du contenant et on enregistre en mémoire les coordonnées des emplacements de ladite ou desdites alvéoles vides dans ledit contenant.

De préférence, la présence d'une ou plusieurs alvéoles vides dans le contenant ainsi mesuré est considérée lors du traitement de la ou des images thermiques acquises par ladite caméra thermique.
Un logiciel, ou programme d'ordinateur, adapté permet lorsqu'il est exécuté par un processeur de traiter la ou les images thermiques ainsi acquises par la caméra thermique pour en déduire à partir de la température calculée de chaque œuf, des informations sur les œufs contenus dans le contenant.
La détection d'alvéoles vides dans le contenant contribue avantageusement à améliorer le traitement statistique des températures et donc d'affiner et rendre ainsi plus fiables les informations obtenues pour chaque œuf présent dans le contenant.

Selon un autre mode de réalisation de ce procédé de contrôle au vol d'œufs placés dans des contenants, pendant ladite étape de mirage des œufs contenus dans ledit contenant, on détermine au moyen d'un capteur de position, la longueur dudit contenant en cours de mesure, on compare la longueur ainsi mesurée dudit contenant avec sa longueur réelle et on en déduit l'absence ou l'existence d'un déplacement intempestif dudit contenant pendant l'étape de mirage.
De manière avantageuse, la conception originale de cette étape autorise une détection simple et peu onéreuse d'un mouvement intempestif d'un contenant transporté par un convoyeur, ce mouvement résultant en une perte de sa position exacte sur le convoyeur pendant une mesure optique des œufs transportés par ce dernier.

Cette étape de détection autorise des cadences extrêmement rapides, typiquement supérieures à 90 000 œufs à l'heure tout en renforçant la fiabilité des mesures de mirage réalisées.

De manière préférentielle, ce capteur de position étant agencé pour détecter les extrémités avant et arrière d'un contenant en déplacement le long de ladite ligne de transport, on mesure l'intervalle de temps séparant la détection par ce capteur desdites extrémités et on calcule une longueur mesurée du contenant par le produit de cet intervalle de temps par la vitesse d'entrainement de ce contenant le long de ladite ligne de traitement.
De manière avantageuse, ce capteur de position est agencé pour détecter ces extrémités d'un contenant lorsqu'elles passent au droit de ce capteur lors du transport du contenant le long de la ligne de traitement.

Alternativement, ce capteur de position étant agencé pour détecter les extrémités avant et arrière d'un contenant en déplacement le long de ladite ligne de transport, on détermine le nombre de points codeur écoulés entre la détection de ces extrémités avant et arrière par ledit capteur et on convertit ce nombre de points codeur en longueur mesurée dudit contenant.
La distance parcourue par la bande du convoyeur pendant un tour codeur étant connue, le nombre de points codeur ainsi déterminé peut aisément être traduit ou converti en distance.
Rappelons que le nombre de points codeur par tour codeur est lié à la résolution de ce codeur.
Le codeur émet avantageusement un signal électrique donnant le nombre de points codeur réalisés entre la détection des deux extrémités avant et arrière.
De manière avantageuse, cette mesure de la longueur du contenant est ainsi indépendante de la vitesse d'entraînement du convoyeur

Lors de l'étape de comparaison, on peut également tenir compte d'une plage de tolérance préalablement déterminée sur la longueur mesurée du contenant.

Selon encore un autre mode de réalisation de ce procédé de contrôle au vol d'œufs placés dans des contenants, on traite également le signal lumineux ayant traversé chaque œuf ainsi détecté pour déterminer la présence de saletés sur les émetteurs de lumière/récepteurs ou les écrans de protection de ces émetteurs/récepteurs, et si tel est le cas, envoyer un signal d'alarme.
De manière avantageuse, avant la mesure d'un nouveau contenant, on réalise avec les émetteurs/récepteurs infrarouge, une mesure de contrôle de la propreté de leur écran transparent de protection à très faible intensité.
Il pourrait également être envisagé de réaliser une détection automatique de présence de saletés sur le système optique de la caméra thermique. Une telle détection serait réalisée par traitement d'au moins une image acquise par cette caméra thermique au moyen d'un logiciel approprié.
Selon un mode de réalisation de ce procédé de détection de présence de saletés sur le système optique de la caméra thermique, on acquiert une ou plusieurs images avec la caméra thermique et on compare chaque image avec des images de référence correspondant à un système optique considéré comme étant propre, ces images de référence étant stockées dans une unité de stockage. Si cette comparaison révèle des différences trop grandes, l'image correspondante est considérée comme étant non fiable et un signal d'alarme est envoyé à l'opérateur pour réaliser une intervention sur la caméra thermique.
Il serait encore possible de réaliser une étape de détection de pollution lumineuse préalablement à une étape de mirage d'un panier. Dans cette étape, une mesure "à vide" (émetteurs éteints) serait effectuée afin de vérifier si les récepteurs reçoivent un signal ou non. Si un tel signal est obtenu alors que les émetteurs IR sont éteints, c'est qu'une autre source IR à 850nm est présente et susceptible de perturber les mesures obtenues lors de l'étape de mirage des œufs contenus dans un panier. Avantageusement, un signal d'alarme est envoyé à l'opérateur.

Des exemples de source lumineuse émettant à 850 nm qui seraient susceptibles de perturber les mesures lors de l'étape de mirage : Lampe halogène, lumière du soleil, ...

Ces mesures de contrôle systématique permettent de vérifier la présence notamment de salissures avant toute mesure d'un nouveau contenant.

Selon encore un autre mode de réalisation de ce procédé de contrôle au vol d'œufs placés dans des contenants, l'acquisition des données et le traitement de ces données sont réalisés en parallèle de sorte que le traitement de l'image ou des images thermiques obtenues pour un premier contenant est réalisé tandis qu'on acquiert une ou plusieurs images thermiques d'un contenant suivant.
De manière avantageuse, un tel traitement en parallèle autorise des cadences plus élevées pour un écartement et une vitesse de défilement des contenants donnés.

Selon encore un autre mode de réalisation de ce procédé de contrôle au vol d'œufs placés dans des contenants, on traite une ou plusieurs images liées à un contenant donné, les informations obtenues par ce traitement étant stockées et/ou adressées à un poste de traitement des œufs distant tel qu'un dispositif d'administration par injection in ovo d'œufs, de sorte que ce poste de traitement recevant ledit contenant d'œufs à traiter dispose des informations nécessaires à son traitement. On assure ainsi une continuité dans le traitement de chaque contenant, le poste de traitement suivant sur la ligne de traitement à hautes cadences ayant déjà reçu de l'appareil de contrôle, les informations relatives au contenant à traiter avant sa prise en charge.

Selon encore un autre mode de réalisation de ce procédé de contrôle au vol d'œufs placés dans des contenants, lesdits contenants sont transportés à une vitesse constante V par un convoyeur rectiligne. Dans la mesure où chaque contenant se déplace en translation, en particulier à vitesse constante sur un convoyeur rectiligne ce qui, à grande cadence, a l'avantage d'éviter les à-coups, les œufs restent stables dans leur alvéole respective du contenant et par conséquent, présentent un positionnement optimal pour leur injection subséquente.
A titre d'exemple, il s'agit d'un convoyeur à bande sans fin.

Selon encore un autre mode de réalisation de ce procédé de contrôle au vol d'œufs placés dans des contenants, lesdits contenants sont transportés à une vitesse strictement supérieure ou égale à 0,11 m/s, et encore mieux à 0,3 m/s, en étant espacés d'une distance de sécurité au moins égale à d = 100 mm pour assurer une cadence de traitement élevée.

Selon encore un autre mode de réalisation de ce procédé de contrôle au vol d'œufs placés dans des contenants, chaque contenant est agencé sur ledit convoyeur rectiligne de sorte que son extrémité avale est perpendiculaire ou sensiblement perpendiculaire au bord latéral du convoyeur rectiligne, une dimension de la matrice de détection de la caméra thermique étant alignée sur cette direction perpendiculaire.

Selon encore un autre mode de réalisation de ce procédé de contrôle au vol d'œufs placés dans des contenants, à partir des données ainsi acquises des œufs contenus dans ledit contenant, on détermine un état de chaque œuf fécondé contenant un embryon.
On cherche alors à déterminer si pour chaque œuf fécondé comportant un embryon, cet embryon est vivant ou mort ou si encore, il est malformé ou trop petit au regard de son âge.
Un tel état étant alors détecté, l'oeuf correspondant sera avantageusement ignoré dans la suite du traitement du contenant, notamment dans l'injection de cet oeuf.

La présente invention concerne également un appareil de contrôle pour la mise en œuvre de différents procédés de contrôle au vol d'œufs placés dans des alvéoles de contenants, et notamment le procédé de contrôle au vol tel que décrit précédemment.
Selon l'invention, cet appareil de contrôle comprend :
- un convoyeur rectiligne pour transporter des contenants d'œufs et déterminant un axe de déplacement de ces contenants,
- au moins une caméra thermique placée de manière fixe le long de l'axe de déplacement des contenants, ladite au moins une caméra thermique étant configurée pour acquérir au moins une image thermique déclenchée par un signal externe,
- un premier capteur de position placé en amont du champ de vision de ladite au moins une caméra thermique et relié à une unité centrale de l'appareil de contrôle de manière à lancer un cycle d'acquisition de données pour un contenant d'œufs dont l'extrémité avale est détectée en une première position définie par ledit premier capteur,
- un ou plusieurs deuxièmes capteurs de position placés en aval de ce premier capteur, ledit ou lesdits deuxièmes capteurs étant reliés à ladite au moins une caméra thermique ou à une unité de contrôle de ladite au moins une caméra thermique pour adresser un signal de déclenchement d'acquisition d'image à ladite ou au moins une desdites caméras thermiques lorsque l'extrémité avale du contenant est détectée en une deuxième position définie par ce ou n'importe quel de ces deuxièmes capteurs de position,
- une pluralité de sources lumineuses pour chacune focaliser un faisceau de lumière dans un œuf à mirer d'un contenant, des détecteurs pour recevoir chacun la lumière passant à travers un œuf ainsi illuminé et une unité de traitement pour traiter le signal lumineux ainsi détecté par chaque détecteur, et
- ladite unité centrale étant configurée pour traiter chacune des images acquises par ladite au moins une caméra thermique.

Un tel appareil permet avantageusement le contrôle au vol, sans contact, d'œufs disposés dans un contenant, ou panier, tout en étant plus sûr pour les embryons des oeufs.

Cet appareil est particulièrement adapté pour le traitement à haute cadence d'objets, sur une ligne automatique industrielle de traitement d'objets ayant un contenu fragile.

Il autorise avantageusement des cadences élevées de traitement automatique typiquement supérieures à 90 000 œufs à l'heure, voire supérieures à 130 000 œufs à l'heure.

L'unité centrale de cet appareil est également configurée pour autoriser un traitement en parallèle des images acquises pour un premier contenant pendant que la caméra thermique acquiert des images pour un second contenant.

Avantageusement, cet appareil de contrôle comporte une unité de commande contrôlant la vitesse de transport des contenants par le convoyeur, laquelle est configurée pour définir une vitesse de transport constante des contenants le long d'un chemin de convoyage.
De manière plus générale, le déplacement des contenants sur le convoyeur rectiligne est réalisé sans à-coups.
Un déplacement à vitesse constante des contenants garantit notamment la stabilité des œufs dans leur alvéole et par conséquent, une orientation optimale de ces œufs pour leur traitement ultérieur sur d'autres postes d'une ligne de traitement à haute cadence.

De préférence, ladite au moins une caméra thermique est configurée de sorte que son champ de vision couvre l'ensemble des alvéoles du contenant selon au moins une première direction de ce dernier.
De manière avantageuse, cette direction est transversale à l'axe de déplacement des contenants.
Les contenants ainsi entraînés par le convoyeur sont espacés d'une distance d telle que le champ de vision de la caméra thermique couvre un nombre entier d'alvéoles selon une seconde direction du contenant, ladite seconde direction étant perpendiculaire à la première direction du contenant.

De préférence, les capteurs de position sont des cellules photoélectriques qui sont, par exemple, placées au-dessus de la bande transporteuse du convoyeur.

De manière avantageuse, les sources lumineuses sont des sources laser équipées de moyens de focalisation pour former un faisceau optique concentré dans leur œuf respectif. A titre purement illustratif, il peut s'agir de diodes lasers (LED) émettant dans l'infrarouge.
De préférence, ces sources lumineuses sont agencées pour former au moins une rangée disposée transversalement à la direction de déplacement des contenants entraînés par le convoyeur de manière à émettre chacune un faisceau optique vers un œuf correspondant d'une même rangée d'un contenant.
Ces éléments sont placés en amont du champ de vision de la caméra thermique le long de l'axe de déplacement des contenants.

De préférence, cette pluralité de sources lumineuses et ces détecteurs sont placés entre le premier capteur de position et ledit au moins un deuxième capteur de position.
Alternativement, ladite pluralité de sources lumineuses et lesdits détecteurs sont placés en aval dudit premier capteur de position et dudit au moins un deuxième capteur de position.

Cet appareil peut également comporter un module de communication pour adresser des données ou informations obtenues par traitement de l'image ou des images d'un contenant donné à un poste distant tel qu'un dispositif d'injection in ovo des œufs de ce contenant.

### Brève description des dessins

D'autres avantages, buts et caractéristiques particulières de la présente invention ressortiront de la description qui va suivre, faite, dans un but explicatif et nullement limitatif, en regard des dessins annexés, dans lesquels :
**Fig. 1**

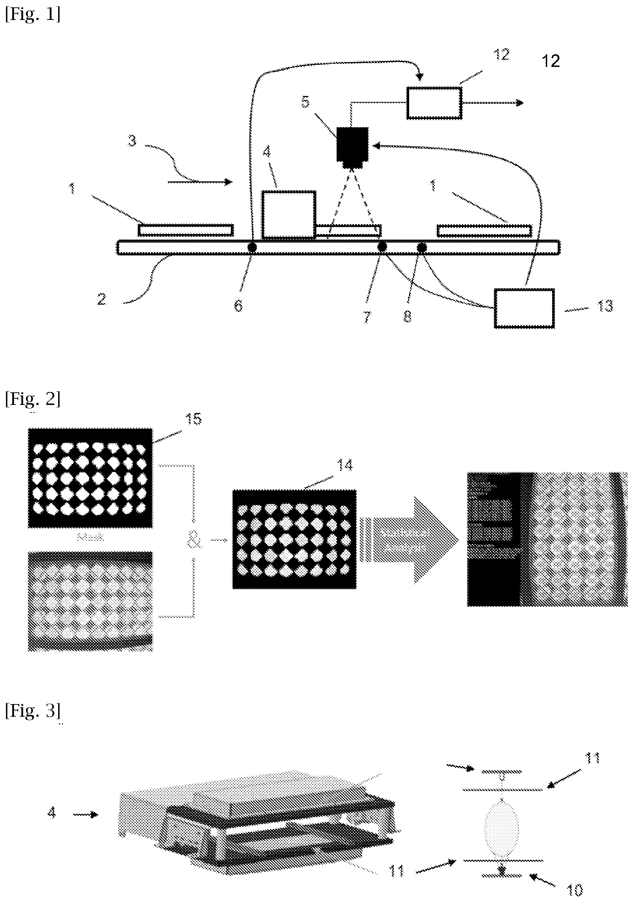

   [Fig. 1] est une représentation schématique partielle d'un appareil pour le contrôle au vol d'œufs disposés dans des paniers en défilement selon un mode de réalisation particulier de la présente invention ;
**Fig. 2**
   [Fig. 2] montre un exemple de traitement de données d'une image acquise avec la caméra thermique de l'appareil de la Fig. 1 ;
**Fig. 3**
   [Fig. 3] est une vue en perspective du dispositif de mirage de l'appareil de contrôle de la Fig. 1 ;

### Description des modes de réalisation

Les dessins et la description ci-après contiennent, pour l'essentiel, des éléments de caractère certain. Ils pourront donc non seulement servir à mieux faire comprendre la présente invention, mais aussi contribuer à sa définition, le cas échéant.

Tout d'abord, on note que les figures ne sont pas à l'échelle.

Les Figures 1 à 3 illustrent de manière schématique un appareil pour le contrôle au vol d'œufs disposés dans des paniers 1 en défilement selon un mode de réalisation particulier de la présente invention

Cet appareil comporte un convoyeur rectiligne 2 pour transporter des paniers 1 suivant un chemin de convoyage définissant un axe de déplacement 3 de ces paniers 1.

Ce convoyeur rectiligne 2 qui est de type à bande sans fin, comporte une unité de commande (non représentée) contrôlant la vitesse de transport des paniers 1.

De manière avantageuse, ces paniers 1 sont déplacés à vitesse constante pour éviter la génération d'à-coups susceptibles d'induire des mouvements des œufs et/ou des chocs aux embryons de ces œufs.

Ces paniers 1 en défilement qui présentent une forme générale "rectangulaire", comprennent une pluralité d'alvéoles, ou cellules, dans chacune desquelles est normalement reçu un œuf.

Ces œufs sont préférablement orientés dans leur alvéole en vue de leur injection in ovo, c'est-à-dire que leur extrémité la plus étroite est disposée vers le bas pour que la chambre à air soit disposée vers le haut. Les risques sont ainsi moindres d'endommager l'embryon de l'oeuf par l'aiguille d'injection. L'œuf est, de préférence, orienté verticalement dans son alvéole.

Les paniers 1 sont alimentés sur le convoyeur rectiligne 2 à intervalle minimal régulier en étant alignés sur une file. Ils présentent ainsi un espacement minimal entre eux.

Le long du chemin de convoyage défini par le convoyeur rectiligne 2 sont disposés de manière fixe, un dispositif de mirage 4, une caméra thermique 5 et une pluralité de capteurs de position 6 - 8.

Le dispositif de mirage 4 comprend des émetteurs 9 et des récepteurs 10 agencés pour assurer le contrôle d'une même rangée d'œufs d'un panier 1. Ces émetteurs 9 et récepteurs 10 sont placés en ligne transversalement à la direction d'avancement des paniers 1. Chaque émetteur 9 est formé d'une diode électroluminescente (LED) émettant à une longueur d'onde de 850 nm. Chaque récepteur 10 est un récepteur infrarouge, lequel possède avantageusement un filtre passe-bande centré autour de la longueur d'onde d'intérêt ici, 850 nm.

De manière connue, ces émetteurs 9 et récepteurs 10 sont protégés des projections et déchets susceptibles de tomber des paniers 1 par des écrans de protection 11 transparents pour la longueur d'onde considérée.

Pour chaque œuf à mirer, un émetteur 9 et un récepteur 10 sont disposés en regard l'un de l'autre, l'émetteur 9 étant placé au-dessus de l'oeuf tandis que le récepteur 10 est placé sous l'oeuf pour recevoir la lumière passant à travers un œuf illuminé par l'émetteur 9 correspondant. Ce dispositif de mirage 4 comporte également une unité de traitement (non représentée) pour traiter le signal lumineux ainsi détecté par chaque récepteur 10.

Ce dispositif de mirage 4 est mis en œuvre pour déterminer les œufs absents dans chaque panier 1 et pour réaliser la détection des œufs non fécondés. C'est un avantage par rapport à l'acquisition des seules images thermiques qui ne permettent pas de différencier les œufs non fécondés des oeufs fertiles morts.

Un premier capteur 6 de position est placé en amont du champ de vision de la caméra thermique 5 et est relié à une unité centrale 12 de manière à lancer un cycle d'acquisition d'images pour chaque panier 1 dont l'extrémité avale est détectée en une position le long de l'axe de déplacement définie par ce premier capteur 6.

On s'assure ainsi que les données qui seront acquises grâce au dispositif de mirage 4 et à la caméra thermique 5 seront bien affectées au panier 1 correspondant, auquel sera attaché un identifiant unique permettant son identification.

Le champ de vision de la caméra thermique 5 recouvre avantageusement la largeur de la bande transporteuse du convoyeur rectiligne 2. Cette caméra thermique 5 à faible temps de réponse permet par son objectif de former une image réelle instantanée de la partie du panier placée dans son champ de vision sur un réseau de cellules photoélectriques. Cette caméra thermique 5 est configurée pour acquérir une image déclenchée par un signal externe (« triggered mode »).

Ces signaux externes sont émis par une carte électronique 12 de contrôle, reliée ici à deux deuxièmes capteurs de position 7-8 placés en aval du premier capteur de position 6 le long du chemin de convoyage. Ces deuxièmes capteurs de position 7-8 sont espacés l'un de l'autre d'une distance d sensiblement égale à la moitié de la longueur du panier à imager.

Pour des contenants plus longs, il peut être nécessaire de disposer le long du chemin de convoyage, trois (3) capteurs de position en aval du premier capteur de position 6. Ces deuxièmes capteurs de position sont alors espacés l'un de l'autre d'une distance d sensiblement égale au tiers de la longueur du panier à imager.

Ainsi un panier 1 est intégralement imagé en deux temps par la caméra thermique 5.

Lorsque l'extrémité avale du panier 1 en cours de cycle d'acquisition est détectée en une position le long du chemin de convoyage définie par un de ces deuxièmes capteurs de position 7-8, un signal de déclenchement est instantanément émis par la carte électronique 13 vers la caméra thermique 5 pour réaliser une prise d'image thermique. La détection de l'extrémité avale du panier 1 par un des deuxièmes capteurs de position 7-8 se traduit par un front montant au niveau du deuxième capteur de position correspondant, lequel déclenche l'envoi du signal à la caméra thermique 5.

L'unité centrale 12 comprend également une unité de traitement pour traiter chacune des images acquises par la caméra thermique 5. De manière avantageuse, cette unité centrale 12 est configurée pour autoriser un traitement en parallèle des images acquises pour un premier panier 1 pendant que la caméra thermique 5 acquiert des images pour un panier 1 suivant.

La combinaison d'une caméra thermique 5 configurée pour acquérir une image suite à la réception d'un signal externe de déclenchement (« triggered mode ») et d'un traitement des images acquises par cette caméra pour un premier panier 1 pendant que de nouvelles images sont acquises pour un panier 1 suivant, autorise avantageusement des cadences élevées de contrôle des œufs, bien supérieures à 90 000 œufs à l'heure.

La Figure 2 illustre un exemple de traitement d'image thermique réalisé par l'unité de traitement de l'unité centrale 12.

Sur chaque image acquise par la caméra thermique 5 pour un panier 1 en cours de cycle d'acquisition, une image corrigée 14 des seuls œufs est générée par application d'un masque 15 permettant d'isoler ces œufs du corps du panier 1 ou encore d'éliminer de l'image thermique brute, le signal lié à ce panier 1.

La position de chaque œuf dans ce panier 1 est ainsi repérée et une analyse statistique de la température de surface et/ou de l'opacité de chacun de ces œufs peut être réalisée.

Les données obtenues par le dispositif de mirage 4 sont utilisées pour tenir compte des signaux liés à des emplacements du panier 1 qui sont vides afin d'améliorer la fiabilité des calculs.

## Revendications

1. Procédé de contrôle au vol d'œufs placés dans des contenants, où
- pendant le déplacement de ces contenants (1) le long d'une ligne de transport, lesdits contenants étant espacés les uns des autres d'au moins une distance de séparation d minimale, on réalise les étapes suivantes:
- on déclenche un cycle d'acquisition de données à chaque passage d'une extrémité avale d'un contenant (1) en une première position, déterminée par un premier capteur de position (6) placé le long de ladite ligne de transport, les positions amont et aval étant considérées dans le sens de déplacement des contenants, puis
- pour un cycle d'acquisition de données d'un contenant (1), on détecte le passage de ladite extrémité avale dudit contenant (1) en au moins une deuxième position déterminée par un deuxième capteur de position (7-8) placé le long de ladite ligne de transport, un signal de déclenchement d'acquisition d'image thermique étant envoyé à une caméra thermique (5) à chaque détection de ladite extrémité avale dudit contenant (1) en au moins une deuxième position pour déclencher une prise d'une ou plusieurs images thermiques par ladite caméra thermique (5) de la partie de contenant (1) alors placée dans son champ de vision,
- lesdits deuxièmes capteurs de position (7-8) étant agencés les uns par rapport aux autres pour assurer un contrôle de l'ensemble des œufs du contenant (1) considéré par ladite caméra thermique (5) lorsque plusieurs deuxièmes capteurs sont mis en œuvre,
- pendant le cycle d'acquisition de données dudit contenant, on réalise également en une troisième position, distincte desdites première et deuxième positions, une étape de mirage des œufs placés dans ledit contenant, selon laquelle on émet un flux lumineux en direction d'au moins un œuf à mirer et on analyse ensuite le flux lumineux passé à travers chaque œuf correspondant en fonction du taux de flux lumineux absorbé par l'oeuf, et
- lesdites données ainsi acquises des œufs d'un contenant (1) étant associées à un identifiant unique de ce contenant (1).

2. Procédé selon la revendication 1, **caractérisé en ce que** ladite étape de mirage est réalisée soit à un instant t₁ à compter de la détection de l'extrémité avale dudit contenant (1) en ladite première position, t₁ étant inférieur au temps nécessaire à l'extrémité avale dudit contenant pour parvenir en ladite au moins une deuxième position, soit par la détection de ladite extrémité avale dudit contenant par un troisième capteur de position placé entre ledit premier capteur de position et ledit au moins un deuxième capteur de position le long de ladite ligne de transport des contenants.

3. Procédé selon la revendication 1, **caractérisé en ce que** ladite étape de mirage est déclenchée par la détection du passage de ladite extrémité avale dudit contenant (1) en une troisième position déterminée par un troisième capteur de position placé en aval du premier capteur de position et dudit au moins un deuxième capteur de position, le long de ladite ligne de transport.

4. Procédé selon l'une quelconque des revendications 1 à 3, **caractérisé en ce que** les œufs étant positionnés en rangées dans des alvéoles dans chaque contenant, on détermine par traitement des signaux obtenus lors de ladite étape de mirage, la ou les alvéoles vides dudit contenant (1) et on enregistre en mémoire les coordonnées des emplacements de ladite ou desdites alvéoles vides dans ledit contenant, la présence d'une ou plusieurs alvéoles vides dans ledit contenant ainsi mesuré étant considérée lors du traitement de la ou des images thermiques acquises par ladite caméra thermique (5).

5. Procédé selon l'une quelconque des revendications précédentes, **caractérisé en ce que** pendant ladite étape de mirage des œufs contenus dans ledit contenant, on détermine au moyen d'un unique capteur de position la longueur dudit contenant en cours de mesure, on compare la longueur ainsi mesurée dudit contenant (1) avec sa longueur réelle et on en déduit l'absence ou l'existence d'un déplacement intempestif dudit contenant pendant l'étape de mirage.

6. Procédé selon la revendication 5, **caractérisé en ce que** ledit capteur de position étant agencé pour détecter les extrémités avant et arrière d'un contenant en déplacement le long dudit axe de déplacement, on mesure l'intervalle de temps séparant la détection par ledit capteur desdites extrémités et on calcule une longueur mesurée dudit contenant par le produit de cet intervalle de temps par la vitesse d'entrainement dudit contenant le long de ladite ligne de transport.

7. Procédé selon l'une quelconque des revendications précédentes, **caractérisé en ce qu'**on traite également le signal lumineux ayant traversé chaque œuf ainsi détecté pour déterminer la présence de saletés sur les émetteurs de lumière/récepteurs ou les écrans de protection de ces émetteurs/récepteurs, et si tel est le cas, envoyer un signal d'alarme.

8. Procédé selon l'une quelconque des revendications précédentes, **caractérisé en ce que** l'acquisition des données et le traitement de ces données sont réalisés en parallèle de sorte que le traitement de l'image ou des images thermiques obtenues pour un premier contenant (1) est réalisé tandis qu'on acquiert une ou plusieurs images thermiques d'un contenant (1) suivant.

9. Procédé selon l'une quelconque des revendications précédentes, **caractérisé en ce que** lesdits contenants sont transportés à une vitesse constante V par un convoyeur rectiligne.

10. Procédé selon l'une quelconque des revendications 1 à 9, **caractérisé en ce qu'**à partir des données ainsi acquises des œufs contenus dans ledit contenant (1), on détermine un état de chaque œuf fécondé contenant un embryon.

11. Appareil pour le contrôle au vol d'œufs placés dans des alvéoles de contenants, **caractérisé en ce qu'**il comprend :
- un convoyeur rectiligne pour transporter des contenants d'œufs et déterminant un axe de déplacement de ces contenants,
- au moins une caméra thermique (5) placée de manière fixe le long de l'axe de déplacement des contenants, ladite au moins une caméra thermique (5) étant configurée pour acquérir au moins une image thermique déclenchée par un signal externe,
- un premier capteur de position (6) placé en amont du champ de vision de ladite au moins une caméra thermique (5) et relié à une unité centrale (12) de l'appareil de contrôle de manière à lancer un cycle d'acquisition de données pour un contenant (1) d'œufs dont l'extrémité avale est détectée en une première position définie par ledit premier capteur,
- un ou plusieurs deuxièmes capteurs de position (7-8) placés en aval de ce premier capteur, ledit ou lesdits deuxièmes capteurs (7-8) étant reliés à ladite au moins une caméra thermique (5) ou à une unité de contrôle de ladite au moins une caméra thermique pour adresser un signal de déclenchement d'acquisition d'image à ladite ou au moins une desdites caméras thermiques (5) lorsque l'extrémité avale du contenant (1) est détectée en une deuxième position définie par ce ou n'importe quel de ces deuxièmes capteurs de position (7-8),
- une pluralité de sources lumineuses (9) pour chacune focaliser un faisceau de lumière dans un œuf à mirer d'un contenant (1), des détecteurs (10) pour recevoir chacun la lumière passant à travers un œuf ainsi illuminé et une unité de traitement pour traiter le signal lumineux ainsi détecté par chaque détecteur, et
- ladite unité centrale (12) étant configurée pour traiter chacune des images acquises par ladite au moins une caméra thermique (5).

12. Appareil selon la revendication 11, **caractérisé en ce que** ladite au moins une caméra thermique (5) est configurée de sorte que son champ de vision couvre l'ensemble des alvéoles du contenant (1) selon au moins une première direction de ce dernier.

13. Appareil selon la revendication 11 ou 12, **caractérisé en ce que** lesdits capteurs de position (6-8) sont des cellules photoélectriques.

14. Appareil selon l'une quelconque des revendications 11 à 13, **caractérisé en ce que** ladite pluralité de sources lumineuses et lesdits détecteurs sont placés entre le premier capteur de position et ledit au moins un deuxième capteur de position.

15. Appareil selon l'une quelconque des revendications 11 à 13, **caractérisé en ce que** ladite pluralité de sources lumineuses et lesdits détecteurs sont placés en aval dudit premier capteur de position et dudit au moins un deuxième capteur de position.

## Patentansprüche

1. Verfahren zur Kontrolle von Eiern in Behältern im laufenden Betrieb, wobei
- während der Bewegung dieser Behälter (1) entlang einer Transportlinie, wobei diese Behälter mindestens einen Mindesttrennabstand d voneinander aufweisen, die folgenden Schritte ausgeführt werden:
- ein Datenerfassungszyklus wird jedes Mal ausgelöst, wenn ein rückwärtiges Ende eines Behälters (1) eine erste Position durchläuft, die durch einen ersten Positionssensor (6) bestimmt wird, der entlang der Transportlinie angeordnet ist, wobei die vorderen und rückwärtigen Positionen in Bewegungsrichtung der Behälter betrachtet werden, dann
- wird für einen Datenerfassungszyklus eines Behälters (1) der Durchlauf des rückwärtigen Endes des Behälters (1) in mindestens einer zweiten Position erkannt, die durch einen zweiten Positionssensor (7-8) bestimmt wird, der entlang der Transportlinie angeordnet ist, wobei jedes Mal, wenn das rückwärtige Ende des Behälters (1) in mindestens einer zweiten Position erkannt wird, ein Auslösesignal für die Wärmebilderfassung an eine Wärmebildkamera (5) gesendet wird, um die Aufnahme eines oder mehrerer Wärmebilder durch die Wärmebildkamera (5) für den Teil des Behälters (1) auszulösen, der sich dann in ihrem Sichtfeld befindet,
- wobei die zweiten Positionssensoren (7-8) relativ zueinander angeordnet sind, um eine Kontrolle aller von der Wärmebildkamera (5) erfassten Eier des Behälters (1) zu gewährleisten, wenn mehrere zweite Sensoren implementiert sind,
- während des Datenerfassungszyklus des Behälters wird außerdem ein Schritt des Durchleuchtens der in den Behälter gelegten Eier in einer dritten Position durchgeführt, die sich von der ersten und zweiten Position unterscheidet, gemäß dem ein Lichtstrom in Richtung von mindestens einem zu durchleuchtenden Ei emittiert wird und der durch jedes entsprechende Ei hindurchtretende Lichtstrom dann in Abhängigkeit von der vom Ei absorbierten Rate des Lichtstroms analysiert wird, und
- wobei die von den Eiern eines Behälters (1) somit gewonnenen Daten einer eindeutigen Kennung dieses Behälters (1) zugeordnet werden.

2. Verfahren nach Anspruch 1, **dadurch gekennzeichnet, dass** der Schritt des Durchleuchtens entweder zu einem Zeitpunkt t₁ ab der Erkennung des rückwärtigen Endes des Behälters (1) in der ersten Position durchgeführt wird, wobei t₁ kürzer ist als die Zeit, die das rückwärtige Ende des Behälters benötigt, um die mindestens eine zweite Position zu erreichen, oder durch Erkennung des rückwärtigen Endes des Behälters durch einen dritten Positionssensor, der zwischen dem ersten Positionssensor und dem mindestens einen zweiten Positionssensor entlang der Behältertransportlinie platziert ist.

3. Verfahren nach Anspruch 1, **dadurch gekennzeichnet, dass** der Schritt des Durchleuchtens durch die Erkennung des Durchlaufs des rückwärtigen Endes des Behälters (1) in einer dritten Position ausgelöst wird, die durch einen dritten Positionssensor bestimmt wird, der dem ersten Positionssensor und dem mindestens einen zweiten Positionssensor entlang der Transportlinie nachgelagert platziert ist.

4. Verfahren nach einem der Ansprüche 1 bis 3,
**dadurch gekennzeichnet, dass,** wobei die Eier in Reihen in Zellen in jedem Behälter angeordnet sind, die leere(n) Zelle(n) des Behälters (1) durch Verarbeitung der während des Durchleuchtungsschritts erhaltenen Signale bestimmt werden und die Koordinaten der Orte der leeren Zelle(n) in dem Behälter im Speicher aufgezeichnet werden, wobei das somit gemessene Vorhandensein einer oder mehrerer leerer Zellen in dem Behälter bei der Verarbeitung des oder der von der Wärmebildkamera (5) aufgenommenen Wärmebildes oder Wärmebilder berücksichtigt wird.

5. Verfahren nach einem der vorstehenden Ansprüche,
**dadurch gekennzeichnet, dass** während des Schritts des Durchleuchtens der in dem Behälter enthaltenen Eier die Länge des Behälters bei der Messung mittels eines einzigen Positionssensors bestimmt wird, die auf diese Weise gemessene Länge des Behälters (1) mit seiner tatsächlichen Länge verglichen wird und daraus auf das Vorliegen oder Nichtvorliegen einer unplanmäßigen Bewegung des Behälters während des Schrittes des Durchleuchtens geschlossen wird.

6. Verfahren nach Anspruch 5, **dadurch gekennzeichnet, dass** der Positionssensor so angeordnet ist, dass er die vorderen und hinteren Enden eines Behälters erkennt, während er sich entlang der Bewegungsachse bewegt, wobei das Zeitintervall zwischen der Erkennung der Enden durch den Sensor gemessen wird und eine gemessene Länge des Behälters durch das Produkt dieses Zeitintervalls mit der Transportgeschwindigkeit des Behälters entlang der Transportlinie berechnet wird.

7. Verfahren nach einem der vorstehenden Ansprüche,
**dadurch gekennzeichnet, dass** das auf diese Weise erkannte Lichtsignal nach dem Hindurchtreten durch jedes Ei ferner verarbeitet wird, um das Vorhandensein von Schmutz auf den Lichtsendern/-empfängern oder den Schutzschirmen dieser Sender/Empfänger zu bestimmen und, falls dies der Fall ist, ein Alarmsignal zu senden.

8. Verfahren nach einem der vorstehenden Ansprüche,
**dadurch gekennzeichnet, dass** die Erfassung der Daten und die Verarbeitung dieser Daten parallel erfolgen, so dass die Verarbeitung des oder der von einem ersten Behälter (1) erhaltenen Wärmebilder erfolgt, während ein oder mehrere Wärmebilder eines folgenden Behälters (1) erfasst werden.

9. Verfahren nach einem der vorstehenden Ansprüche,
**dadurch gekennzeichnet, dass** die Behälter mit einer konstanten Geschwindigkeit V von einem geraden Förderband transportiert werden.

10. Verfahren nach einem der Ansprüche 1 bis 9,
**dadurch gekennzeichnet, dass** anhand der auf diese Weise von den in dem Behälter (1) enthaltenen Eiern erfassten Daten der Zustand jedes befruchteten Eies, das einen Embryo enthält, bestimmt wird.

11. Vorrichtung zur Kontrolle von in Zellen der Behälter abgelegten Eiern im laufenden Betrieb, **dadurch gekennzeichnet, dass** sie beinhaltet:
- ein gerades Förderband zum Transportieren von Eierbehältern und zum Bestimmen einer Bewegungsachse dieser Behälter,
- mindestens eine Wärmebildkamera (5), die stationär entlang der Bewegungsachse der Behälter angeordnet ist, wobei die mindestens eine Wärmebildkamera (5) so konfiguriert ist, dass sie, ausgelöst durch ein externes Signal, mindestens ein Wärmebild aufnimmt,
- einen ersten Positionssensor (6), der vor dem Sichtfeld der mindestens einen Wärmebildkamera (5) platziert und mit einer Zentraleinheit (12) der Steuervorrichtung verbunden ist, um einen Datenerfassungszyklus für einen Behälter (1) mit Eiern zu starten, dessen rückwärtiges Ende in einer ersten, durch den ersten Sensor definierten Position erkannt wird,
- einen oder mehrere zweite Positionssensoren (7-8), die diesem ersten Sensor nachgelagert platziert sind, wobei der oder die zweiten Sensoren (7-8) mit der mindestens einen Wärmebildkamera (5) oder mit einer Steuereinheit der mindestens einen Wärmebildkamera verbunden sind, um ein Bilderfassungs-Auslösesignal an die oder mindestens eine der Wärmebildkameras (5) zu senden, wenn das rückwärtige Ende des Behälters (1) in einer zweiten Position erkannt wird, die durch diesen oder einen beliebigen dieser zweiten Positionssensoren (7-8) definiert ist,
- eine Vielzahl von Lichtquellen (9), von denen jede einen Lichtstrahl auf ein zu durchleuchtendes Ei in einem Behälter (1) fokussiert, Detektoren (10), von denen jeder das Licht empfängt, das durch ein somit beleuchtetes Ei hindurchtritt, und eine Verarbeitungseinheit zum Verarbeiten des somit von jedem Detektor erkannten Lichtsignals, und
- wobei die Zentraleinheit (12) dazu konfiguriert ist, jedes der von der mindestens einen Wärmebildkamera (5) erfassten Bilder zu verarbeiten.

12. Vorrichtung nach Anspruch 11, **dadurch gekennzeichnet, dass** die mindestens eine Wärmebildkamera (5) so konfiguriert ist, dass ihr Sichtfeld sämtliche Zellen des Behälters (1) in mindestens einer ersten Richtung von diesem abdeckt.

13. Vorrichtung nach Anspruch 11 oder 12, **dadurch gekennzeichnet, dass** die Positionssensoren (6-8) fotoelektrische Zellen sind.

14. Vorrichtung nach einem der Ansprüche 11 bis 13,
**dadurch gekennzeichnet, dass** die Vielzahl von Lichtquellen und die Detektoren zwischen dem ersten Positionssensor und dem mindestens einen zweiten Positionssensor platziert sind.

15. Vorrichtung nach einem der Ansprüche 11 bis 13,
**dadurch gekennzeichnet, dass** die Vielzahl von Lichtquellen und die Detektoren dem ersten Positionssensors und dem mindestens einen zweiten Positionssensors nachgelagert platziert sind.

## Claims

1. Method for inspecting, as they pass, eggs placed in containers, wherein
- during the movement of these containers (1) along a conveyor line, said containers being spaced apart from each other by at least a minimum separation distance d, the following steps are carried out:
- triggering a data acquisition cycle on each passage of a downstream end of a container (1) at a first position, which is determined by a first position sensor (6) placed along said conveyor line, upstream and downstream positions being considered with reference to the direction of movement of the containers; then
- for a data acquisition cycle of a container (1), detecting the passage of said downstream end of said container (1) at at least a second position determined by a second position sensor (7-8) placed along said conveyor line, a signal triggering thermal image acquisition being sent to a thermal camera (5) each time said downstream end of said container (1) is detected at at least one second position in order to trigger capture of one or more thermal images by said thermal camera (5) of the portion of the container (1) placed in its field of view,
- said second position sensors (7-8) being arranged with respect to one another to ensure an inspection of all of the eggs of the container (1) considered by said thermal camera (5) when a plurality of second sensors are employed,
- the data acquisition cycle of said container comprises performing, in a third position separate from said first and second positions, a step of candling the eggs placed in said container, in which step a light flux is emitted in the direction of at least one egg to be candled and the light flux passed through each corresponding egg is then analyzed depending on the level of light flux absorbed by the egg, and
- said data thus acquired on the eggs of a container (1) being associated with a unique identifier of this container (1).

2. Method according to claim 1, **characterized in that** said candling step is carried out either at an instant t₁ from the detection of the downstream end of said container (1) at said first position, t₁ being less than the time required for the downstream end of said container to reach said at least one second position, or by the detection of said downstream end of said container by a third position sensor placed between said first position sensor and said at least one second position sensor along said container conveyor line.

3. Method according to claim 1, **characterized in that** said candling step is triggered by the detection of the passage of said downstream end of said container (1) in a third position determined by a third position sensor placed downstream of the first position sensor and of said at least one second position sensor, along said conveyor line.

4. Method according to any one of claims 1 to 3, **characterized in that** the eggs being positioned in rows in divots within each container, the empty divot(s) of said container (1) are determined by processing the signals obtained during said candling step and the coordinates of the locations of said empty divot(s) in said container are saved in memory, the presence of one or more empty divots in said container thus measured being considered during the processing of the thermal image(s) acquired by said thermal camera (5).

5. Method according to any one of the preceding claims,
**characterized in that** during said step of candling the eggs contained in said container, the length of said container being measured is determined by means of a single position sensor, the thus measured length of said container (1) is compared with its real length and the absence or existence of an inadvertent movement of said container during the candling step is deduced therefrom.

6. Method according to claim 5, **characterized in that** said position sensor being arranged to detect the front and rear ends of a container moving along said axis of movement, the time interval separating the detection by said sensor of said ends is measured and a measured length of the container is calculated by the product of this time interval multiplied by the driving speed of said container along said conveyor line.

7. Method according to any one of the preceding claims, **characterized in that** the light signal that passed through each egg thus detected is also treated to determine the presence of grime on the light emitters/receivers, or the protective screens of these emitters/receivers, and if any is present, to send an alarm signal.

8. Method according to any one of the preceding claims,
**characterized in that** the acquisition of the data and the processing of these data are carried out in parallel so that the processing of the image or of the thermal images obtained for a first container (1) is carried out while one or more thermal images of a subsequent container (1) are being acquired.

9. Method according to any one of the preceding claims, **characterized in that** said containers are transported at a constant speed V by a straight conveyor.

10. Method according to any one of claims 1 to 9, **characterized in that** from the data thus acquired from the eggs contained in said container (1), a state of each fertilized egg containing an embryo is determined.

11. Apparatus for inspecting, as they pass, eggs placed in the divots of containers, **characterized in that** it comprises:
- a straight conveyor for transporting containers of eggs and determining an axis of movement of these containers,
- at least one thermal camera (5) placed fixedly along the axis of movement of the containers, said at least one thermal camera (5) being configured to acquire at least one thermal image triggered by an external signal,
- a first position sensor (6) placed upstream of the field of view of said at least one thermal camera (5) and connected to a central processing unit (12) of the inspection apparatus so as to initiate a data acquisition cycle for an egg container (1), the downstream end of which is detected in a first position defined by said first sensor,
- one or more second position sensors (7-8) placed downstream of this first sensor, said second sensor or said second sensors (7-8) being connected to said at least one thermal camera (5) or to a control unit of said at least one thermal camera to send a signal triggering image acquisition to said or at least one of said thermal cameras (5) when the downstream end of the container (1) is detected in a second position defined by that or any of these second position sensors (7-8),
- a plurality of light sources (9) for each focus of a light beam in an egg to be candled in a container (1), detectors (10) for each receiving the light passing through an egg thus illuminated and a processing unit for processing the light signal thus detected by each detector, and
- said central unit (12) being configured to process each of the images acquired by said at least one thermal camera (5).

12. Apparatus according to claim 11, **characterized in that** said at least one thermal camera (5) is configured so that its field of view covers all of the divots of the container (1) according to at least one first direction thereof.

13. Apparatus according to claim 11 or 12, **characterized in that** said position sensors (6-8) are photoelectric cells.

14. Apparatus according to any one of claims 11 to 13, **characterized in that** said plurality of light sources and said detectors are placed between the first position sensor and said at least one second position sensor.

15. Apparatus according to any one of claims 11 to 13, **characterized in that** said plurality of light sources and said detectors are placed downstream of said first position sensor and of said at least one second position sensor.
